(19) Europäisches Patentamt
European Patent Office
Office européen des brevets

(11) **EP 3 628 368 A1**

(12) **EUROPÄISCHE PATENTANMELDUNG**

(43) Veröffentlichungstag:
**01.04.2020   Patentblatt 2020/14**

(51) Int Cl.:
***A61N 1/37*** *(2006.01)*

(21) Anmeldenummer: **19207935.8**

(22) Anmeldetag: **29.11.2010**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2009   US 28885809 P**

(62) Dokumentnummer(n) der früheren Anmeldung(en) nach Art. 76 EPÜ:
**10192894.3 / 2 338 566**

(71) Anmelder: **BIOTRONIK SE & Co. KG**
**12359 Berlin (DE)**

(72) Erfinder:
• **Dörr, Thomas**
**12437 Berlin (DE)**

• **Weiss, Ingo**
**12435 Berlin (DE)**

(74) Vertreter: **Galander, Marcus**
**Biotronik Corporate Services SE**
**Corporate Intellectual Property**
**Sieversufer 7 - 9**
**12359 Berlin (DE)**

Bemerkungen:
Diese Anmeldung ist am 08.11.2019 als Teilanmeldung zu der unter INID-Code 62 erwähnten Anmeldung eingereicht worden.

(54) **IMPLANTIERBARES MEDIZINISCHES GERÄT MIT MITTELN ZUR REKONSTRUKTION EINES UNVOLLSTÄNDIG ERFASSTEN SIGNALS**

(57)    Die vorliegende Erfindung beschreibt ein implantierbares medizinisches Gerät (IMD), mit:
- einer Einheit zur Erkennung von elektromagnetischen Störungen, beinhaltend einen Sensor oder Indikator für elektromagnetische Störfelder und mindestens einen Zeitmesser,
- einer Steuereinheit, die mit der Einheit zur Erkennung von elektromagnetischen Störungen verbindbar ist, und
- einer Elektrodenleitung, die mit der Steuereinheit verbunden ist und die am anderen Ende eine Elektrode aufweist,
- wobei die Steuereinheit ausgebildet ist, eine Rekonstruktion von über die mindestens eine Elektrode erfassten elektrischen Messungen für die Zeitfenster durchführt, in denen elektromagnetische Störungen erkannt werden.

100

110        120        130

**FIG. 1**

EP 3 628 368 A1

**Beschreibung**

Die Erfindung bezieht sich auf eine Vorrichtung und ein Verfahren zum Umgang mit elektromagnetischen Feldern, speziell solchen Feldern, wie sie bei bildgebenden Kernspintomographie- (im folgenden MRT- oder MRI-) Geräten auftreten.

Obwohl MRI-Untersuchungen einen immer höheren Stellenwert in der diagnostischen Medizin erhalten, ist ein Teil der Patienten für MRI-Untersuchungen kontraindiziert. Eine solche Kontraindizierung kann durch ein mindestens teilweise implantiertes medizinisches Gerät (im Folgenden auch Implantat oder IMD) gegeben sein. Neben den MRI-Untersuchungen stellen aber auch andere technische Anwendungen eine Gefahr für die Nutzer von medizinischen Geräten oder implantierbaren medizinischen Geräten dar, besonders, wenn diese starke elektromagnetische Störfelder (EMI, Electro Magnetic Interference) in Ihrer Umgebung erzeugen.

Um MRI-Untersuchungen dennoch zu ermöglichen, sind verschiedene Ansätze, die sich entweder auf die Durchführung der MRI-Untersuchung oder auf das implantierbare medizinische Gerät beziehen, bekannt.

Unter anderem sind Technologien zur Erkennung von Magnetfeldern bekannt, die auf herkömmlichen Verfahren zur Magnetfelddetektion beruhen. So beschreibt die US 2008/0154342 ein Verfahren unter Ausnutzung eines GMR-Sensors (Giant Magnetic Resistance), um problematische Magnetfelder von MRT-Geräten zu erkennen. Diese technologischen Ansätze sind allerdings wenig spezifisch und erzeugen einen erhöhten Energiebedarf, was zu einer kürzeren Betriebsdauer bei gleichen Energiereserven führt.

US 20060293591 A1 beschreibt ein implantierbares medizinisches Gerät mit einer Telemetrie-Antenne und einem Lead mit einem länglichen Körper umfassend einen Leiter, der sich von einem proximalen Anschluss zu einer distalen Elektrode erstreckt.

US 20040263172 A1 beschreibt eine Spannungskompensationseinheit, welche durch induzierte Spannungen verursachte Effekte auf ein Gerät mit einem einzelnen Leiter reduziert.

Auch ist aus dem Stand der Technik die Schrift US 2005/0070787 bekannt, die eine Vorrichtung zum koordinierten Abschalten von bestimmten Komponenten eines implantierten medizinischen Gerätes während der Abgabe von elektromagnetischen Strahlungsimpulsen beschreibt. Dazu ist einen Kommunikation vom MRI-Gerät zum Implantat notwendig, bei der Informationen zum zeitlichen Ablauf vom MRI-Gerät zum Implantat übertragen werden. Bisher ist eine solche Kommunikation zwischen Implantat und MRI-Geräten nicht vorgesehen, so dass ein Einsatz erst mit einer Aufrüstung von MRI-Geräten und Implantaten oder zumindest den MRI-Geräten möglich ist. Des Weiteren stellt der Stand der Technik keine weiteren Lösungen bereit, die die abgeschalteten Komponenten ersetzt, womit eine angepasste Arbeitsweise eines implantierten medizinischen Gerätes und/oder eine ständige Überwachung von physiologischen Parametern eines Implantatträgers nicht in jedem Fall gewährleistet ist. Zusätzlich ist der Stand der Technik durch die notwendige Kommunikation zwischen Implantat und MRI-Gerät angewiesen, wodurch andere Störquellen nicht erkannt und im Betrieb berücksichtigt werden können.

Daher ist es Aufgabe der Erfindung, die Mängel im Stand der Technik zu beheben und ein Gerät bereitzustellen, das eine hohe Zuverlässigkeit unter elektromagnetischen Störungen (EMI, Electro Magnetic Interference) ermöglicht.

Die Aufgabe wird durch ein zumindest teilweise implantierbares medizinisches Gerät (IMD) mit den Merkmalen des Anspruchs 1 gelöst.

Dabei beinhaltete das zumindest teilweise implantierte medizinisches Gerät (IMD) mindestens eine Einheit zur Erkennung von elektromagnetischen Störungen, mindestens beinhaltend einen Sensor oder Indikator für elektromagnetische Störfelder und mindestens einen Zeitmesser, mindestens eine Steuereinheit, die mit der Einheit zur Erkennung von elektromagnetischen Störungen verbindbar ist, mindestens eine Elektrodenleitung, die mit der Steuereinheit verbunden ist und die am anderen Ende eine Elektrode aufweist, die im Kontakt mit Körpergewebe ist und wobei die Elektrode sich entweder ins Körperinnere erstreckt oder auf der Oberfläche eines Implantates befindet, wobei die Steuereinheit die Abgabe von elektrischen Stimulationsimpulsen über die mindestens eine Elektrode nur in Zeitfenstern erlaubt, in denen keine elektromagnetischen Störungen erkannt werden und/oder die Steuereinheit eine Rekonstruktion von über die mindestens eine Elektrode erfassten elektrischen Messungen für die Zeitfenster durchführt, in denen elektromagnetische Störungen erkannt werden.

Unter die Begrifflichkeit mindestens teilweise implantiert fallen dabei sowohl vollständig implantierte Systeme, als auch solche, bei denen nur Komponenten, zum Beispiel, aber nicht beschränkt auf, Elektroden, inklusive Teile der Elektrodenleitungen und/oder optische Leiter, mit Sensoren und/oder Aktuatoren und/oder Stimulatoren, und/oder Leitungen von internen oder externen Medikamentenpumpen und/oder Flüssigkeitspumpen zur Diagnose und/oder Therapie. Die Flüssigkeitspumpen können einerseits geeignet sein, Flüssigkeiten aus dem Körperinneren zu einer Analyseeinheit und/oder Therapieeinheit zu befördern, die sich sowohl innerhalb, als auch außerhalb des Körpers befinden kann.

Bevorzugt wird, dass das zumindest teilweise implantierbare medizinische Gerät ein externer Herzschrittmacher und/oder externer Defibrillator oder implantierter Herzschrittmacher und/oder Defibrillator/Cardioverter und/oder ein kardiales Resynchronisationstherapiegerät (CRT), ein Herzunterstützungssystem, wie eine Herzunterstützungspumpe, ein künstliches Herz, ein Neurostimulator, ein implantiertes Überwachungsgerät (Patientenmonitor), eine implantierte Medikamentenpumpe, eine implantierte Flüssigkeitspumpe, und/oder ein externes Herz-Lungen-System ist, und dass die Steuereinheit des IMD den Betriebszustand des IMD anhand der detektierten elektromagnetischen Störung und/oder von mit der mindestens einen Elektrode detektierten Messwerten und/oder von rekonstruierten Messwerten aus vorbestimmbaren Betriebszuständen auswählt.

Auch wird bevorzugt, dass der Zeitmesser einen Speicher aufweist, der ausgelegt ist, Zeitdifferenzen, wie Therapieintervalle und/oder Intervalle von intrinsischen Systemereignissen, zu speichern. Unter Therapieintervallen sind dabei die Intervalle zu verstehen, in denen Therapien abgegeben werden, wobei sowohl einzelne Therapien, als auch Gruppen von Therapien umfasst sind. Therapieintervall sind beispielsweise, aber nicht beschränkt auf, RR- oder QT- oder TQ-Zeit oder ein entsprechendes Parametertupel aus einem IEGM. Auch bei den Intervallen von intrinsischen Systemereignissen sind verschiedene Intervalle möglich, wie, aber nicht beschränkt auf einzelne Systemereignisse und/oder Gruppen von Systemereignissen, wie QRS- oder andere Komplexe oder komplette Herzzyklen und/oder krankhafte Ereignisse. Unter IEGM versteht der Fachmann im Allgemeinen ein intrakardiales Elektrokardiogramm.

Besonders bevorzugt wird, dass der zeitliche Abstand zwischen zwei elektrischen Stimuli ein Therapieintervall ist und dass zumindest das jeweils vorhergehende Therapieintervall speicherbar ist.

Auch wird besonders bevorzugt, dass der Speicher als "first in first out" (fifo) organisiert ist.

Bevorzugt wird auch, dass die Steuereinheit mit den Daten des Zeitmessers den Zeitpunkt der nächsten Therapie berechnet.

Besonders bevorzugt wird, dass die Berechnung des nächsten Therapiezeitpunktes auf einer Herzrhythmusschätzung und/oder einem Herzsimulator beruht. Parameter für die Modelle der Berechnung der Therapiezeitpunkte sind programmierbar und/oder die Modelle zur Berechnung der Therapiezeitpunkte die IEGM- und/oder EKG- und/oder rekonstruierten IEGM Messungen zur Berechnung heranziehen, wobei EKG-Daten über externe Hilfsmittel ermittelt werden und dem zumindest teilweise implantierten medizinischen Gerät zur Verfügung gestellt werden und auch Daten aus der Patientenhistorie aus den vorhanden Informationsquellen, wie, aber nicht darauf beschränkt, Patientenakten, Patientendatenbanken und/oder Patientengerät, und/oder die Berechnung auf den im Implantat gespeicherten Parameter und Messungen beruht, speziell aus einem Langzeitspeicher und/oder die Berechnung auf den im Implantat gespeicherten Parameter und Messungen beruht, bei deren Aufnahme keine elektromagnetischen Störungen detektiert worden sind.

Auch wird besonders bevorzugt, dass die Berechnung auf einem Approximationsmodell der kardialen Restitution und/oder einem linearen oder nicht linearen Zeitreihenmodell und/oder auf einem gewichteten Mittel von n vorangegangenen Zeitdifferenzen, mit n größer oder gleich 1, beruht.

Unter kardialer Restitution versteht der Fachmann den Zusammenhang zwischen der Aktionspotentialdauer von Herzmuskelzellen in Abhängigkeit der Dauer des vorangegangenen diastolischen Intervalls - graphisch dargestellt auch als Restitutionskurve bekannt. Umgekehrt, bei Kenntnis des diastolischen Intervalls kann mit Hilfe der Restitutionskurve die Repolarisationszeit des nächstfolgenden Herzzyklus geschätzt werden und damit die Stimulation in die vulnerable Phase vermieden werden. Die Restitutionskurve kann in abgeänderter Form auch in Abhängigkeit der vorhergehenden RR Intervalls angegeben werden. Im einfachsten Fall bildet die Kurve nur den statischen Zusammenhang zwischen Herzrate und Repolarisationsverhalten ab. Ein Modellansatz höherer Detailtiefe berücksichtigt auch die dynamischen Zusammenhänge, d. h die Repolarisationszeit hängt auch von der weiteren Vergangenheit der Herzaktivität ab (z. B. aber nicht beschränkt auf mehrerer vorangegangenen Herzratenintervalle sowie mehrerer vorangegangenen Repolarisationszeiten), wie insbesondere berücksichtigt in dieser Erfindung. Man spricht hier von einer Gedächtnistiefe. So lautet in einfachster Darstellung das Restitutionsgesetz:

$$RmTm = f(Rm\text{-}1Rm)$$

Oder allgemeiner

$RmTm = f([Rm\text{-}i\text{-}1Rm\text{-}i],[Rm\text{-}i\text{-}1Tm\text{-}i\text{-}i],[p])$; i=0..N, N=Gedächtnistiefe, p=Parameter-vektor der diese Gesetzmäßigkeit parametriert

Des Weiteren wird bevorzugt, dass bei der Berechnung des nächsten Therapiezeitpunktes die vulnerablen Phasen des Herzerregungszyklus vermieden werden und/oder Parameter für die Modelle der Berechnung der Therapiezeitpunkte programmierbar sind und/oder die Modelle zur Berechnung der Therapiezeitpunkte die IEGM- und/oder EKG- und/oder rekonstruierten IEGM Messungen zur Berechnung heranziehen, wobei EKG-Daten über externe Hilfsmittel ermittelt werden und dem zumindest teilweise implantierten medizinischen Gerät zur Verfügung gestellt werden und auch Daten aus der Patientenhistorie aus den vorhandenen Informationsquellen einbezogen werden, wie Patientenakten, Patientendatenbanken, und/oder Patientengerät, und/oder die Berechnung auf den im Implantat gespeicherten Parametern und Messungen beruht, speziell aus einem Langzeitspeicher und/oder die Berechnung auf den im Implantat gespeicherten Parametern und Messungen beruht, bei deren Aufnahme keine elektromagnetischen Störungen detektiert worden sind. Besonders relevant ist diese Ausführung im Zusammenhang mit dem so genannten Home Monitoring, bei dem in vorbestimmbaren Abständen Daten aus einem Implantat an eine zentrale Stelle gesendet werden und von dort oder auch direkt einem Arzt zur Verfügung gestellt werden. Mit diesem Ausführungsbeispiel kann sichergestellt werden, dass nur valide Daten an die Patientendatenbank, bzw. den Arzt weitergeleitet werden.

Es wird auch bevorzugt, dass die Stimulationseinheit triggerbar und/oder retriggerbar ist, speziell durch Messungen in Zeitfenstern ohne detektierte elektromagnetische Störfelder und/oder durch rekonstruierte Messdaten zwischen den Zeitfenstern ohne detektierte elektromagnetische Störfelder, und/oder die Steuereinheit die Stimulationsabgabe unterdrückt oder eine sub-threshold oder zero-intensity Stimulationsabgabe auslöst, wenn ein intrinsisches Ereignis von der Detektionseinheit der Steuereinheit detektiert wurde und/oder durch die Rekonstruktion von Detektionslücken identifiziert wurde. Mit sub-threshold intensity sind Stimulationsimpulse gemeint, die unterhalb einer vorbestimmbaren und/oder voreinstellbaren Reizschwelle liegen, während die zero-intensity Stimulationen mit einer nicht vorhandenen Intensität ausgeführt oder nur getriggert werden.

Besonders bevorzugt wird, dass die Steuereinheit eine Prioritätsschaltung besitzt, die eine Therapie auch in Zeitfenstern temporär erlaubt, in denen elektromagnetische Störungen von der Einheit zur Erkennung von elektromagnetischen Störungen detektiert worden sind, wobei die Prioritätsschaltung immer aktivierbar ist und/oder nur aktivierbar ist, wenn eine elektromagnetische Störung von der Einheit zur Erkennung von elektromagnetischen Störungen detektiert worden ist und die Störung unter mindestens einen vorbestimmbaren Sensormesswert und/oder mindestens einem vorbestimmbaren Indikatorwert liegt. In diesem Zusammenhang ist "vorbestimmbar" auch so zu verstehen, dass einer von mehreren vorbestimmten Sensormesswerten und/oder vorbestimmten Indikatorwerten in Abhängigkeit vom Patientenzustand Anwendung finden.

Ebenfalls wird bevorzugt, dass die Rekonstruktion der Messdaten zwischen den Zeitfenstern ohne eine detektierte elektromagnetische Störung mindestens eine der folgenden Eigenschaften aufweist, die Detektionseinheit verfügt zusätzlich Blankingeinheit, die Überschwinger in detektierten Messsignalen ausblendet, wobei die Blankingeinheit das Relaxationsverhalten des Systems, wie das System Elektrode und Eingangsschaltkreis, und/oder die Beschaffenheit der Störung, wie hochfrequente Störungen wie von RF-Feldern, niederfrequente Störungen, wie von Gradientenfeldern, berücksichtigt, für die Rekonstruktion wird je nach Länge der Störung eine Rekonstruktionsmethode automatisch ausgewählt wird, wie lineare oder polynominale Interpolation, wobei die Ableitungen an den Anschlussstellen an die Messwerte stetig sind, für kurze Störlängen und eine Mustererkennung von typischen Messkurven, wie QRS-Komplexe und/oder T-Wellen und/oder p-Wellen und/oder Kombinationen wie QRS-T-Abschnitt und/oder T-P-Abschnitt und/oder P-QRS-Abschnitt, für die Rekonstruktion eine Mustererkennung verwendet wird, die typische Messkurven erkennt und die erkannten Muster an die Steuereinheit weiterleitet, so dass die erkannten Muster, wie QRS-Komplexe und/oder T-Wellen und/oder p-Wellen und/oder Kombinationen wie QRS-T-Abschnitt und/oder T-P-Abschnitt und/oder P-QRS-Abschnitt, für ein Triggern und/oder Retriggern der Stimulationseinheit verwendbar sind, und die Rekonstruktion mittels einer Mustererkennung bei mittleren oder langen Störstellen auf Basis der erkannten Muster, wie aber nicht beschränkt auf QRS-Komplexe und/oder T-Wellen und/oder p-Wellen und/oder Kombinationen wie QRS-T-Abschnitt und/oder T-P-Abschnitt und/oder P-QRS-Abschnitt, ein Ergänzungsmuster ermittelt.

Auch wird bevorzugt, dass das IMD in einen von einer Signaldetektion unabhängigen Betriebszustand geschaltet wird, wenn keine Signalrekonstruktion aus den detektierten Signalen möglich ist, wobei das unter anderem, aber nicht beschränkt auf, bei kurzen Zeitfenstern ohne elektromagnetische Störungen in Kombination mit mindestens mittellangen Zeiträumen mit elektromagnetischen Störungen auftritt.

Des Weiteren wird bevorzugt, dass mindestens eine der folgenden Maßnahmen bei einer Erkennung von elektromagnetischen Störungen eingeleitet wird, das Wechseln in einen MRI-Sicherenzustand, ein verlängertes Verbleiben in einem MRI-sicheren oder gegenüber elektromagnetischen Störfeldern unempfindlichen Zustand, und die Abgabe von elektromagnetischen Pulsen zur Signalisierung, dass ein medizinisches Gerät, speziell ein Implantat, im elektromagnetischen Feld vorhanden ist, speziell zur Signalisierung an ein MRI-Gerät, mit der Möglichkeit neben der Störung auch Informationen auf diese Weise zu übertragen und auf dem Bildschirm des MRI sichtbar zu machen.

Ebenfalls wird bevorzugt, dass die Einheit zur Erkennung von elektromagnetischen Störungen zur Detektion von elektromagnetischen Feldern mindestens einen der folgenden Sensoren oder Indikatoren umfasst, GMR-Sensor, MagFET-Sensor, Hallsensor, elektrooptische Wandler als Indikator, die Überwachung von Batteriespannungen während Kondensatorladeprozessen als Indikator, die Detektion von RF-Feldern als Indikator, die Detektion von magnetischen Grandientenfeldern als Indikator, die Detektion von durch elektromagnetische Felder induzierten Strömen als Indikator, die Detektion von spezifischen Vibrationen oder als Sensoren ausgelegte Bauteile zur Detektion von durch Lorentzkräfte induzierten Vibrationen als Indikator.

Beschreibung der Figuren:

Einige Aspekte der Erfindung werden in den Figuren 1 bis 7 dargestellt.

Fig. 1    schematische Darstellung des Ablaufs einer MRI-Untersuchung

Fig. 2 schematische Darstellung der Vorgehensweise in Bezug auf Rekonstruktion und/oder Interpolation

Fig. 3 schematische Darstellung einer Rekonstruktion und/oder Interpolation einer IEGM Abtastlücke

Fig. 4 mögliche Implementierung

Fig. 5 RT Prädiktor

Fig. 6 wichtige Regeln

Fig. 7 Verzögerung um Sicherheitsabstand

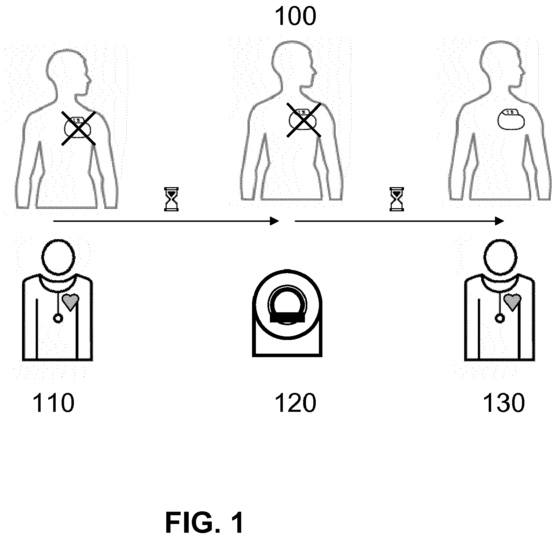

Figur 1 beschreibt den Stand der Technik, bei dem der ICD-Patient (100) vor der geplanten MRT-Untersuchung von einem Kardiologen nachgesorgt und der ICD ausgeschaltet (110) wird. Mit einer zeitlichen Verzögerung von Stunden bis Tagen erfolgt die MRT-Untersuchung bei einem Radiologen (120). Nach einer weiteren Verzögerung wird der Patient wieder vom Kardiologen (130) betreut und der ICD wieder eingeschaltet. Während der gesamten Zeit von (110) bis (130) ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung, in Kauf genommen.

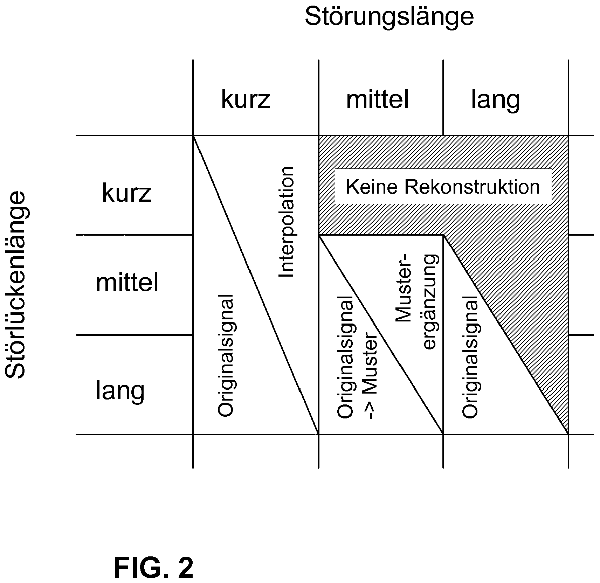

Die Figur 2 zeigt eine mögliche Interpretationsmatrix für mögliche Reaktionen für unterschiedliche Verhältnisse von Störlückenlänge und Störungslänge, wobei die Störlückenlänge die Zeitabschnitte ohne Störungen bezeichnet und die Störlänge die Länge der auftretenden und/oder wahrgenommenen elektromagnetischen Störungen ist. Die Art der Erkennung der elektromagnetischen Störungen ist für die angepasste IEGM-Abtastung nicht von Bedeutung, prinzipiell kommen alle zur Erkennung von elektromagnetischen Störungen geeigneten Technologien in Frage, besonders, aber nicht beschränkt auf diese, GMR-Sensoren, MagFET-Sensoren, Hallsensoren, elektrooptische Wandler, die Überwachung von Batteriespannungen während Kondensatorladeprozessen, die Detektion von RF-Feldern, die Detektion von magnetischen Grandientenfeldern, die Detektion von durch elektromagnetische Felder induzierten Strömen, die Detektion von spezifischen Vibrationen oder als Sensoren ausgelegte Bauteile zur Detektion von durch Lorentzkräfte induzierten Vibrationen. Die Matrix gibt beispielhaft an, bei welchen Verhältnissen von Störlückenlänge zur Störungslänge rekonstruiert, interpoliert und/oder das Originalsignal verwendet wird.

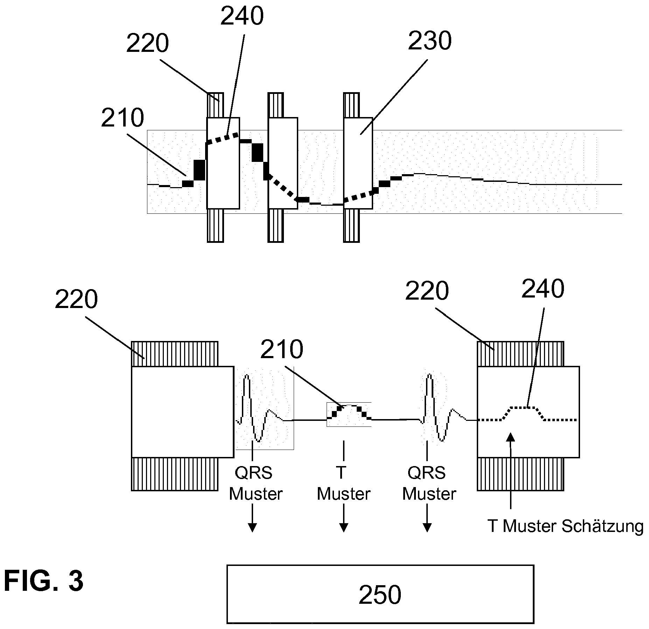

Die Figur 3 zeigt eine beispielhafte Rekonstruktion des Originalsignals 210 während der Störungen. Das Originalsignal 210 wird in der oberen Darstellung von einer Störung 220 überlagert, wodurch ein Blankingfenster 230 entsteht, das heißt ein Fenster, in dem keine Signale detektiert werden können, das über die Dauer der Störung 220 hinausreicht. In dem Blankingfenster 240 wird das Signal durch Interpolation rekonstruiert, so dass ein rekonstruierter Abschnitt 240 entsteht.

In der unteren Darstellung in Figur 3 wird das gemessene Originalsignal 210 an einen Herzsimulator 250 weitergeleitet und so die während des Blankingfensters 230 entstehende Lücke durch die Störung 220 im Originalsignal 210 von dem Herzsimulator 250 mit der Rekonstruktion 240 aufgefüllt. Die Rekonstruktion ist dem gewählten Beispiel eine Schätzung des T-Musters. Mit dieser oder der oben zu Figur 3 beschriebenen Vorgehensweise lassen sich Abtastlücken in einem IEGM füllen.

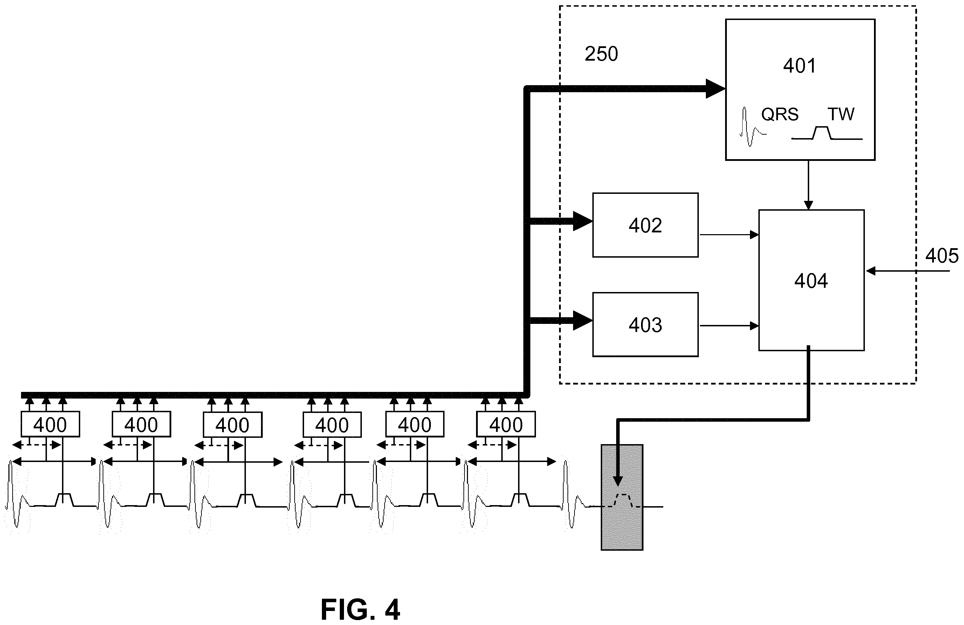

Eine mögliche Implementierung beschreibt Figur 4. Metrik-Extraktoren 400 vermessen laufend oder durch einen Trigger wie z. B. vom Störfelddetektor ausgelöst über Signal 405 Herzzyklen hinsichtlich RR Intervallen, RT Intervallen, QRS-Amplitude, -breite, -Fläche ferner T-Wellen-Amplitude, -breite, -Fläche, sowie Morphologien, aber nicht beschränkt auf diese. Diese sind Eingangsinformationen für den Herzsimulator 250, in einer erfindungsgemäß besonders bevorzugten Implementierung bestehend aus einer Morphologiedatenbank 401, einem RR-Prädiktor 402 und RT-Prädiktor 403. Passend zur Vorgeschichte (beispielhaft Gedächtnistiefe 6) wird das nächstfolgende RR und RT Intervall geschätzt, damit dieses im Falle einer kommenden Störung sofort verfügbar sind. Ebenso bereitet die Datenbank aufgrund der Eingangsinformation die Morphologien vor (als normierte Kurven für z. B. QRS oder T-Wellen Rekonstruktion), die bei ähnlicher vorangegangener Morphologieabfolge in der Vergangenheit schon mal diesem Muster gefolgt hat (im Sinne der höchsten Wahrscheinlichkeit). Mit den Ergebnissen des RR- und RT-Prädiktors wird dieses Muster dann skaliert und positioniert. Dies realisiert der Rekonstruktor 404.

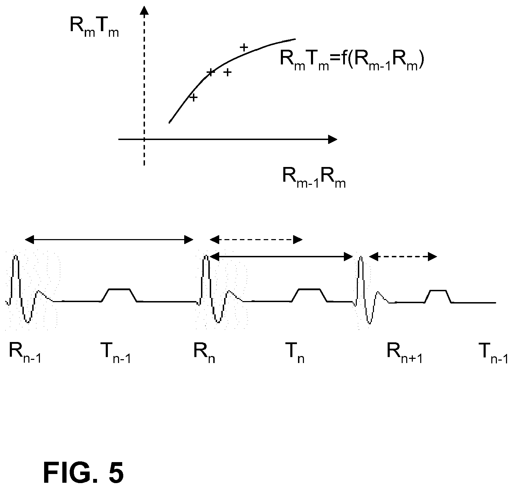

Figur 5 erläutert den RT-Prädiktor. Jedes, entsprechend der Gedächtnistiefe, RT Intervall wird als Ordinate über dem vorhergehenden RR Intervall als Abszisse aufgetragen. Gemäß der Gesetzmäßigkeit wird die aktuell gültige Restitution Kurve gefittet (z. B. Methode der kleinsten Fehlerquadrate). Damit kann das RT Intervall des nächstfolgenden Herzzyklus bestimmt werden. Dieser Vorgang kann als sliding window oder blockweise realisiert sein.

Der RR-Prädiktor (nicht dargestellt) kann z. B. als Zeitreihenmodell realisiert sein, mit folgender Berechungsvorschrift.

$$R_n R_{n+1} = \sum_{i=1}^{N} a_i R_{n-i} R_{n-i+1}$$

Eine erweiterte Realisierung berücksichtigt außer RR Intervallen auch RT Intervalle, QRS-Amplitude, -breite, -Fläche ferner T-Wellen-Amplitude, -breite, -Fläche, sowie Morphologien (z. B. Morphologieklassen und/oder -indizes wie z. B. definiert in der US 2006/0047216A1, aber nicht beschränkt auf diese). Ebenso sieht eine weitere erweiterte Realisierung auch nichtlineare Verknüpfungen der Eingangsinformationen vor.

In einer besonders bevorzugten Realisierung wählt der Rekonstruktor die Morphologie des zu schätzenden Signalabschnittes (z. B. QRS-Komplex oder T-Welle etc) aus der Datenbank und skaliert und positioniert diese auf der Zeitachse hinein in den fehlenden Signalabschnitt gemäß den geschätzten RT und RR Intervallwerten. Die Amplitudenskalierung erfolgt aufgrund von Verhältnismäßigkeiten die aus vergangenen Signalabschnitten erlernt wurden. So z. B. wird die Amplitude der rekonstruierten T-Welle prozentual zur davor liegenden R-Zacke skaliert, ggf. auch unter Berücksichtigung von Mittelwerten aus vorherigen R-Zacken sowie vorherigen T-Wellenamplituden.

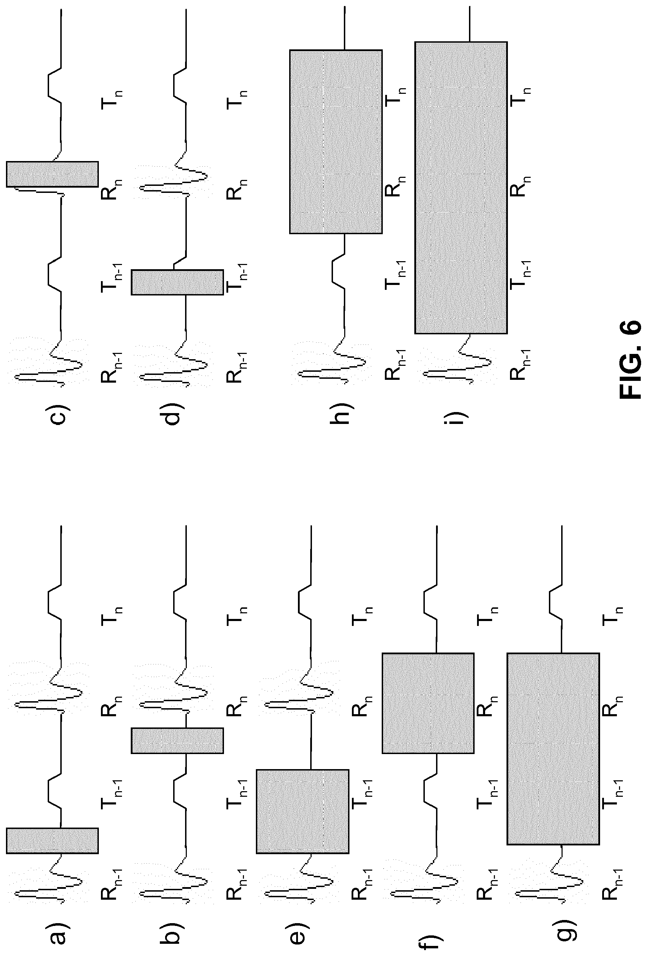

Illustriert mit Figur 6 werden im Folgenden die wichtigsten Regeln beschrieben, die in der besonders bevorzugten Implementierung des Rekonstruktors umgesetzt sind (aber nicht beschränkt hierauf). Das Blankingsignal 405 definiert die zu rekonstruierenden Bereiche:

In den Fällen a) und b) sind nur kurze Signalstücke auf isoelektrischen Linien des IEGMs gestört. Hier wird der fehlende Signalabschnitt durch eine Gerade die den letzten Punkt vor der Störung mit dem ersten Punkt nach der Störung verbindet ersetzt. Eine weitere Lösung sieht eine Kurvenlinie geringster Ordnung vor, deren erste Ableitung an den Kontaktpunkten stetig ist.

Die Fälle c) und d) zeigen Beispiele, wo ein QRS oder T-Wellen Abschnitt nur teilweise gestört ist. Die werden durch den Rekonstruktor ergänzt mit einem Muster, das bei ähnlicher Signalvorgeschichte an dieser Stelle am wahrscheinlichsten folgen würde.

Im Fall e) schätzt der RT-Prädiktor die RT-Zeit, die T-Wellen-Morphologie wird aus der Datenbank gewählt, die bei ähnlicher Signalvorgeschichte an dieser Stelle am wahrscheinlichsten folgen würde.

Im Fall f) schätzt der RR-Prädiktor die RR-Zeit, die QRS-Morphologie wird aus der Datenbank gewählt, die bei ähnlicher Signalvorgeschichte an dieser Stelle am wahrscheinlichsten folgen würde.

Im Fall g) werden die folgenden T- und QRS-Signale geschätzt. In der Implementierung in der der RR-Prädiktor das vorangehende RT Intervall auch benötigt wird dafür der vom RT-Prädiktor geschätzte Wert genommen.

In den Fällen f) und g) wird in einer besonders bevorzugten Realisierung der fehlende Zeitpunkt Rn nicht nur vom RR-Prädiktor berechnet, sondern zusätzlich aus der Restitutionskurve bestimmt und zwar aus der Lösung des nichtlinearen Gleichungssystems:

$$Rn\text{-}1Rn + RnTn = b$$

$$RnTn = f(Rn\text{-}1Rn)$$

Dabei ist b bekannt, da das Signal zu den Zeitpunkten Rn-1 und Tn nicht gestört ist.

Die beiden Ergebnisse für Rn-1Rn erhalten durch den RR-Prädiktor einerseits und aufgrund der oben genannten Methode andererseits werden in einer weiterhin bevorzugten Implementierung zu einem Mittelwert verknüpft der dann den gültigen Zeitpunkt Rn ergibt.

Im Fall h) wird zunächst der nächste QRS geschätzt. Dieses RR Intervall nutzt dann der RT-Prädiktor, die nächste RT-Zeit zu schätzen.

Im Fall i) setzt sich das wie in den Fällen g und h fort. Je länger der gestörte Signalabschnitt ist, desto ungenauer wird die Rekonstruktion, daher wird ab einer voreinstellbaren Störlänge in einen Modus umgeschaltet, der den Patienten therapeutisch versorgt, ohne das IEGM Signal zu benötigen. Dies ist in einer bevorzugten Implementierung eine festfrequente Stimulation, z. B. D00, V00 oder A00 Mode.

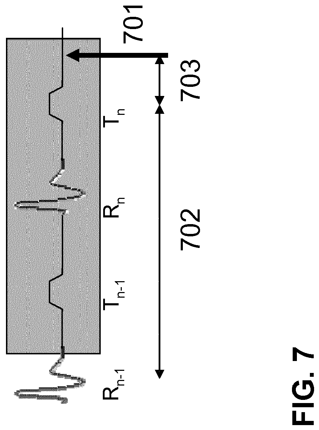

Dauert die Störung lange an, kann nicht mehr sicher festgestellt werden, ob eine Herzaktivität vorliegt. Erfindungsgemäß ist daher nach Ablauf des watchdog-Zählers 702 ein Sicherheitsstimulus 701 vorgesehen. Unter Berücksichtigung des RR und RT-Prädiktors wird dabei die Lage der vulnerablen Phase geschätzt, in die nicht hineinstimuliert werden darf, da dies sonst eine Arrhythmie auslösen könnte. Daher wird nach Ablauf des watchdog-Zählers ggf. die Abgabe dieses Stimulus weiter verzögert, um einen Sicherheitsabstand 703 (Fig. 7).

**Patentansprüche**

1. Zumindest teilweise implantierbares medizinisches Gerät (IMD), mit:

   - mindestens einer Einheit zur Erkennung von elektromagnetischen Störungen (220), mindestens beinhaltend einen Sensor oder Indikator für elektromagnetische Störfelder und mindestens einen Zeitmesser,
   - mindestens einer Steuereinheit die mit der Einheit zur Erkennung von elektromagnetischen Störungen (220) verbindbar ist,
   - mindestens einer Elektrodenleitung, die mit der Steuereinheit verbunden ist und die am anderen Ende eine Elektrode aufweist, wobei

   die Steuereinheit ausgebildet ist, eine Rekonstruktion von über die mindestens eine Elektrode erfassten elektrischen Messungen für die Zeitfenster durchführt, in denen elektromagnetische Störungen (220) erkannt werden,

die Steuereinheit ausgebildet ist, die Abgabe von elektrischen Stimulationsimpulsen über die mindestens eine Elektrode nur in Zeitfenstern erlaubt, in denen keine elektromagnetischen Störungen erkannt werden und/oder die Steuereinheit eine Rekonstruktion von über die mindestens eine Elektrode erfassten elektrischen Messungen für die Zeitfenster durchführt, in denen elektromagnetische Störungen erkannt werden, wobei
**gekennzeichnet dadurch, dass**
die Steuereinheit ausgebildet ist, die Abgabe von elektrischen Stimulationsimpulsen über die mindestens eine Elektrode nur in Zeitfenstern zu erlauben, in denen keine elektromagnetischen Störungen (220) erkannt werden, wobei die Steuereinheit ausgebildet ist, mit den Daten des Zeitmessers den Zeitpunkt der nächsten Therapie zu berechnen. wobei die Berechnung des nächsten Therapiezeitpunktes auf einer Herzrhythmusschätzung und/oder einem Herzsimulator beruht. automatisch auszuwählen.

2.  IMD nach Anspruch 1, **dadurch gekennzeichnet, dass** das zumindest teilweise implantierbare medizinische Gerät:

    - ein externer Herzschrittmacher oder externer Defibrillator oder implantierter Herzschrittmacher oder Defibrillator/Cardioverter oder ein kardiales Resynchronisationstherapiegerät (CRT),
    - ein Herzunterstützungssystem, wie eine Herzunterstützungspumpe,
    - ein künstliches Herz,
    - ein Neurostimulator,
    - ein implantiertes Überwachungsgerät (Patientenmonitor),
    - eine implantierte Medikamentenpumpe,
    - eine implantierte Flüssigkeitspumpe, und/oder
    - ein externes Herz-Lungen-System ist,

    und dass die Steuereinheit des IMD den Betriebszustand des IMD anhand der detektierten elektromagnetischen Störung und/oder von mit der mindestens einen Elektrode detektierten Messwerten und/oder von rekonstruierten Messwerten aus vorbestimmbaren Betriebszuständen auswählt.

3.  IMD nach einem der vorangegangenen Ansprüche, **dadurch gekennzeichnet, dass** der Zeitmesser einen Speicher aufweist, der ausgelegt ist, Zeitdifferenzen, wie Therapieintervalle und/oder Intervalle von intrinsischen Systemereignissen, zu speichern.

4.  IMD nach Anspruch 3, **dadurch gekennzeichnet, dass** die Steuereinheit ausgebildet ist, zumindest das jeweils vorhergehende Therapieintervall im Speicher zu speichern.

5.  IMD nach einem der Ansprüche 3 oder 4, **dadurch gekennzeichnet, dass** der Speicher als "first in first out" (fifo) organisiert ist.

6.  IMD nach einem der vorigen Ansprüche, **dadurch gekennzeichnet, dass** die Berechnung auf einem Approximationsmodell der kardialen Restitution und/oder einem linearen oder nicht linearen Zeitreihenmodell und/oder auf einem gewichteten Mittel von n vorangegangenen Zeitdifferenzen, mit n größer oder gleich 1, beruht.

7.  IMD nach einem der vorigen Ansprüche, **dadurch gekennzeichnet, dass** die Steuereinheit ausgebildet ist, bei der Berechnung des nächsten Therapiezeitpunktes die vulnerablen Phasen des Herzerregungszyklus zu vermeiden und/oder
Parameter für die Modelle der Berechnung der Therapiezeitpunkte programmierbar sind und/oder
die Modelle zur Berechnung der Therapiezeitpunkte die IEGM- und/oder EKG- und/oder rekonstruierten IEGM Messungen zur Berechnung herangezogen werden, wobei EKG-Daten über externe Hilfsmittel ermittelt werden und dem zumindest teilweise implantierten medizinischen Gerät zur Verfügung gestellt werden und auch Daten aus der Patientenhistorie aus den vorhandenen Informationsquellen einbezogen werden, wie Patientenakten, Patientendatenbanken und/oder Patientengerät, und/oder
die Berechnung auf den im IMD gespeicherten Parametern und Messungen beruht, und/oder
die Berechnung auf den im Implantat gespeicherten Parametern und Messungen beruht, bei deren Aufnahme keine elektromagnetischen Störungen (220) detektiert worden sind.

8.  IMD nach einem der vorangegangenen Ansprüche, **dadurch gekennzeichnet, dass**:

    die Stimulationseinheit ausgebildet ist, triggerbar und/oder retriggerbar zu sein, speziell durch Messungen in Zeitfenstern ohne detektierte elektromagnetische Störfelder und/oder durch rekonstruierte Messdaten zwischen den Zeitfenstern ohne detektierte elektromagnetische Störfelder, und/oder
    die Steuereinheit ausgebildet ist, die Stimulationsabgabe zu unterdrücken oder eine subthreshold oder zero-Intensity Stimulationsabgabe auszulösen, wenn ein intrinsisches Ereignis von einer Detektionseinheit der Steuereinheit detektiert wurde und/oder durch die Rekonstruktion von Detektionslücken identifiziert wurde.

9.  IMD nach Anspruch 8, **dadurch gekennzeichnet,**

**dass** die Steuereinheit eine Prioritätsschaltung besitzt, wobei die Prioritätsschaltung ausgelegt ist, eine Therapie auch in Zeitfenstern temporär zu erlauben, in denen elektromagnetische Störungen (220) von der Einheit zur Erkennung von elektromagnetischen Störungen (220) detektiert worden sind, wobei die Prioritätsschaltung immer aktivierbar ist, wenn eine elektromagnetische Störung von der Einheit zur Erkennung von elektromagnetischen Störungen (220) detektiert worden ist und die Störung unter mindestens einen vorbestimmbaren Sensormesswert und/oder mindestens einen vorbestimmbaren Indikatorwert fällt.

10. IMD nach einem der vorangegangenen Ansprüche, **dadurch gekennzeichnet, dass** die Steuereinheit ausgebildet ist, die Rekonstruktion der Messdaten zwischen den Zeitfenstern ohne eine detektierte elektromagnetische Störung mit mindestens einer der folgenden Eigenschaften durchzuführen:

die Detektionseinheit verfügt zusätzlich über eine Blankingeinheit, die Überschwinger in detektierten Messsignalen ausblendet,
für die Rekonstruktion wird je nach Länge der Störung eine Rekonstruktionsmethode automatisch ausgewählt,
für die Rekonstruktion eine Mustererkennung verwendet wird, oder
die Rekonstruktion mittels einer Mustererkennung bei mittleren oder langen Störstellen auf Basis der erkannten Muster ein Ergänzungsmuster ermittelt.

11. IMD nach einem der vorangegangenen Ansprüche, **dadurch gekennzeichnet, dass** das IMD dazu ausgelegt ist, in einen von einer Signaldetektion unabhängigen Betriebszustand zu schalten, wenn keine Signalrekonstruktion aus den detektierten Signalen möglich ist.

12. IMD nach einem der vorangegangenen Ansprüche, **dadurch gekennzeichnet, dass** das IMD dazu ausgelegt ist, mindestens eine der folgenden Maßnahmen bei einer Erkennung von elektromagnetischen Störungen (220) einzuleiten:

das Wechseln in einen MRI-sicheren Zustand,
ein verlängertes Verbleiben in einem MRI-sicheren oder gegenüber elektromagnetischen Störfeldern unempfindlichen Zustand, und
die Abgabe von elektromagnetischen Pulsen zur Signalisierung, dass ein medizinisches Gerät, speziell ein Implantat, im elektromagnetischen Feld vorhanden ist.

13. IMD nach einem der vorangegangenen Ansprüche, **dadurch gekennzeichnet, dass** die Einheit zur Erkennung von elektromagnetischen Störungen (220) zur Detektion von elektromagnetischen Feldern mindestens einen der folgenden Sensoren oder Indikatoren umfasst:

- GMR-Sensor,
- MagFET-Sensor,
- Hallsensor,
- elektrooptische Wandler als Indikator,
- die Überwachung von Batteriespannungen während Kondensatorladeprozessen als Indikator,
- die Detektion von RF-Feldern als Indikator,
- die Detektion von magnetischen Grandientenfeldern als Indikator,
- die Detektion von durch elektromagnetische Felder induzierten Strömen als Indikator,
- die Detektion von spezifischen Vibrationen oder als Sensoren ausgelegte Bauteile zur Detektion von durch Lorentzkräfte induzierten Vibrationen als Indikator.

**FIG. 1**

EP 3 628 368 A1

**FIG. 2**

QRS
Muster

T
Muster

QRS
Muster

T Muster Schätzung

FIG. 3

250

**FIG. 4**

$R_m T_m$

$R_m T_m = f(R_{m-1} R_m)$

$R_{m-1} R_m$

$R_{n-1}$ $T_{n-1}$ $R_n$ $T_n$ $R_{n+1}$ $T_{n-1}$

FIG. 5

**FIG. 6**

EP 3 628 368 A1

FIG. 7

Europäisches Patentamt
European Patent Office
Office européen des brevets

# EUROPÄISCHER RECHERCHENBERICHT

**Nummer der Anmeldung**

EP 19 20 7935

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2006/293591 A1 (WAHLSTRAND JOHN D [US] ET AL) 28. Dezember 2006 (2006-12-28) * Absatz [0013] - Absatz [0021] * * Absatz [0025] * * Absatz [0032] - Absatz [0039] * * Absatz [0048] - Absatz [0051]; Abbildungen 1, 2, 5 * ----- | 1-13 | INV. A61N1/37 |
| Y | US 2004/263172 A1 (GRAY ROBERT W [US] ET AL) 30. Dezember 2004 (2004-12-30) * Absatz [0084] - Absatz [0085] * * Absatz [0097] - Absatz [0109]; Abbildungen 1, 3, 6 * ----- | 1,2,5-13 | |
| Y | US 2003/144705 A1 (FUNKE HERMANN D [BE]) 31. Juli 2003 (2003-07-31) * Absatz [0047] - Absatz [0057] * * Absatz [0072] - Absatz [0082]; Abbildungen 1, 3 * ----- | 1-13 | |
| Y | US 2004/193224 A1 (HEDBERG SVEN ERIK [SE] ET AL) 30. September 2004 (2004-09-30) * Absatz [0008] - Absatz [0012] * * Absatz [0020] - Absatz [0025]; Abbildungen 1, 3-5 * ----- | 10 | RECHERCHIERTE SACHGEBIETE (IPC) A61N |

Der vorliegende Recherchenbericht wurde für alle Patentansprüche erstellt

| Recherchenort | Abschlußdatum der Recherche | Prüfer |
|---|---|---|
| München | 13. Februar 2020 | Aronsson, Fredrik |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 20 7935

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-02-2020

| Im Recherchenbericht angeführtes Patentdokument | Datum der Veröffentlichung | Mitglied(er) der Patentfamilie | Datum der Veröffentlichung |
|---|---|---|---|
| US 2006293591 A1 | 28-12-2006 | US 2006293591 A1 | 28-12-2006 |
| | | WO 2006124481 A2 | 23-11-2006 |
| US 2004263172 A1 | 30-12-2004 | CA 2517771 A1 | 13-01-2005 |
| | | EP 1636612 A2 | 22-03-2006 |
| | | JP 2007524458 A | 30-08-2007 |
| | | US 2004263172 A1 | 30-12-2004 |
| | | US 2004263173 A1 | 30-12-2004 |
| | | US 2004263174 A1 | 30-12-2004 |
| | | US 2008058913 A1 | 06-03-2008 |
| | | WO 2005003790 A2 | 13-01-2005 |
| US 2003144705 A1 | 31-07-2003 | CA 2485398 A1 | 20-11-2003 |
| | | EP 1506037 A1 | 16-02-2005 |
| | | JP 4297872 B2 | 15-07-2009 |
| | | JP 2005525179 A | 25-08-2005 |
| | | US 2003144705 A1 | 31-07-2003 |
| | | WO 03095022 A2 | 20-11-2003 |
| US 2004193224 A1 | 30-09-2004 | DE 60302868 T2 | 20-07-2006 |
| | | EP 1464360 A1 | 06-10-2004 |
| | | US 2004193224 A1 | 30-09-2004 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080154342 A
- US 20060293591 A1
- US 20040263172 A1
- US 20050070787 A
- US 20060047216 A1